Europäisches Patentamt

European Patent Office

Office européen des brevets

(11) Publication number: **0 261 884**
**B1**

# EUROPEAN PATENT SPECIFICATION

(45) Date of publication of the patent specification:
29.08.90

(51) Int. Cl.⁵: **A61F 2/76, A61F 2/60**

(21) Application number: **87308274.7**

(22) Date of filing: **18.09.87**

(54) Limb prosthesis production and material therefor.

(30) Priority: **18.09.86 GB 8622454**

(43) Date of publication of application:
**30.03.88 Bulletin 88/13**

(45) Publication of the grant of the patent:
**29.08.90 Bulletin 90/35**

(84) Designated Contracting States:
**DE ES FR GB IT**

(56) References cited:
**US-A- 2 424 278**
**US-A- 2 664 572**
**US-A- 3 393 407**

**COMPUTER SYSTEMS, vol. 6, no. 10, October 1986,**
**pages 15,16, Bromley, GB; "Shape a leg"**

(73) Proprietor: **NATIONAL RESEARCH DEVELOPMENT**
**CORPORATION, 101 Newington Causeway, London**
**SE1 6BU(GB)**

(72) Inventor: **Causton, Brian Edward, 10 Church Road,**
**Aldermaston Reading Berkshire(GB)**

(74) Representative: **Parker, Geoffrey, Patent Department**
**National Research Development**
**Corporation 101 Newington Causeway, London**
**SE1 6BU(GB)**

ACTORUM AG

## Description

This invention has been conceived and developed primarily in relation to a particular form of limb prosthesis and it is both convenient and appropriate to describe the invention in this particular context. However, it will be appreciated that the invention is equally applicable to other forms of limb prostheses where similar circumstances prevail.

The particular prosthesis in question is the so-called Patella Tendon Borne form which is widely used for below-the-knee amputee patients. The success of such a prosthesis rests heavily on the quality of the fit of a custom made stump cup in the prosthesis into which the limb stump is to be received. Clearly if the cup is too small the prosthesis is unacceptable. Difficulty also arises if, in compensation to avoid undersizing, the cup is too big. This difficulty arises from the fact that it is normally intended for the patient's body weight in the relevant limb to be taken on the patella tendon and the calf but, if the cup is oversized, the stump can sink into the cup to subject the end of the bone to load. This is very painful, as are pressures on the front of the tibia and on the protuberances at the sides of the condyles, and also on areas of tissue overlying major nerves, particularly where those nerves have been severed during surgery.

The usual mode of production with a view to obtaining a good fit for the cup is referred to as the wrap cast technique because it entails first taking an accurate impression of the stump by wrapping a plaster bandage therearound to form a cast. During this wrapping, and while the plaster is setting, the soft tissue of the stump is manipulated by the prosthetist into an acceptable disposition for the purposes of subsequent use of the prosthesis, and this operation is most important in determining the final anatomical shape of the cup. After the cast is set, it is used as a mould in which a replica of the manipulated stump is cast. The cup is then made up on this replica, but only after the replica has been rectified in order that the cup is enlarged relative to the original replica in appropriate areas whereby the bone end and other sensitive parts of the stump will not be subjected to undesirable pressure.

The disadvantage of this technique arises from the fact that the rectification is a lengthy process, typically of about three hours duration, and it is often effected remotely of the patient at a factory rather than by the prosthetist at the hospital or limb fitting centre.

In an alternative mode of production referred to as the pressure casting technique, the prosthetist can both take castings and rectify in the same procedure. In this case clay is built up on the stump in the areas to be relieved from pressure in the cup, and then a complex pressure jacket is applied to the limb and an impression of the stump taken. This technique is time consuming, complicated, user-sensitive, and not in common usage.

An object of the present invention is to ameliorate the difficulties of this situation.

In one aspect of the invention there is provided a method of making a limb prosthesis for an amputee patient which comprises rectifying the limb stump by the application thereto of at least one moulding of viscoelastic material precast to a predetermined shape and then taking an impression of the rectified stump, which impression determines the shape of a stump-receiving cup in said prosthesis.

This aspect of the invention rests on the appreciation that the application of clay in use of the pressure casting technique follows long established rectification principles whereby substantially standard shapes are built up. In the present case the applied material is precast in such a shape rather than built up and it can be selected from a range of sizes for a given shape and/or from a range of different shapes to suit different circumstances.

In another aspect the invention provides an assembly of separate mouldings of viscoelastic material individually precast to a respective predetermined shape for use in the method of the invention.

The material of the invention is to be substantially stable in use, at least to the extent that it does not flow from its appointed position during manipulation by the prosthetist. Also the material should be sufficiently flexible to allow underlying tissue to be manipulated during wrap casting. Preferably in addition the material should adhere well to skin, without causing irritation, or to a suitable protective film material wrapped around the stump, and such adhesion should not break down during wrap casting. At the same time the material should preferably not adhere to, nor inhibit the setting of, plaster bandage or other material used in forming a rectified cast, although difficulty in this respect may be obviated by using an outer protective layer of a suitable film material.

These properties can be met by a variety of materials such as plasticised polyvinylchloride, butyl rubber, natural rubber latex foam and silicone rubber. Any rubber in fact can be used, as can synthetic polymer hydrogels providing that plaster accelerators are also used to prevent setting inhibition.

It has been preferred in development of the invention to date to employ a gel which fulfils all of the above requirements while being additionally advantageous in terms of economy of cost and ease of fabrication.

This last material is made by adding a mixture of plasticiser, dibutyl phthalate, and ethanol to beads of poly(ethylmethacrylate). The result is a liquid which is mobile for about 5 minutes whereafter gelation becomes apparent and is complete in about 20 minutes. The rate of gelation is controllable by way of the alcohol/plasticiser ratio and the molecular weight of the polymer, and the stiffness of the final product material is controllable by way of the polymer/liquid ratio.

In one specific suitable example PEM of molecular weight 40,000 was used as beads of diameter less than 150μm. The related liquid comprised 15% ethanol and 85% phthalate, and the polymer/ liquid ratio was 100 ml to 100 g.

Many different plasticisers can be used, as can other methacrylates and related polymers such as acrylates. However. among the methacrylates the methyl form is not suitable.

In production of the proposed assemblies from such material it is preferred that the final mixture be poured, before gelation, into moulds in the form of suitably shaped depressions representing the desired range and formed in spaced but compact distribution in a tray of polythene or other readily formable material from which the mouldings can be peeled for use. The tray is suitably of a stackable form whereby a number thereof can be packaged for storage and/or distribution without the precast shapes being subjected to prolonged force which may cause distortion.

Further clarification of the invention can be gained by consideration of examples of the associated assembly and method described with reference to the accompanying drawings, in which:-

Figures 1, 2 and 3 illustrate, respectively in plan view and related cross-sectional views taken at II-II and III-III, an assembly for use in the production of a patella tendon borne prosthesis, and

Figures 4-9 which schematically illustrate use of the illustrated assembly.

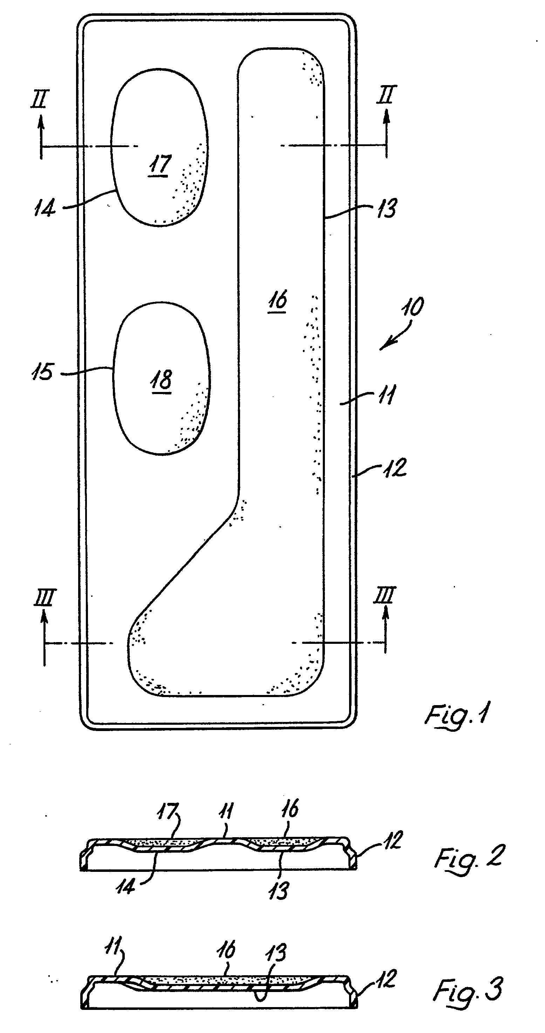

The assembly of Figures 1-3 comprises a tray 10 of suitable material and composed of a rectangular platform 11 having a downwardly turned rim 12 around its periphery. The platform is formed with three dished zones 13-15 in which viscoelastic material of the above-mentioned gel kind is cast up to the top surface level of the platform, the resultant mouldings being denoted respectively 16-18. The overall depth of the dished zones is no more than half that of the tray rim, and the rim is outwardly stepped at or just below the depth of the zones so that the assembly is stackable with others of like form and with a clearance between the zones, from one tray to the next.

The zones 13-15 involve two different shapes. Zone 13 conforms to a first one of these shapes, which shape is seen in plan as that of a strip having one end portion transversely extended along one side, with the extension preferably being of generally progressively increasing width towards the adjacent strip end. The other shape for zones 14 and 15 is that of an oval disc as seen in plan. Each shape has a cross-sectional shaping which is decreasingly tapered towards its periphery to give a flared form.

Insofar as the mouldings are to be applied to prescribed different areas of the same limb and the proportions of a given limb are generally uniform from one person to another, the mould shapes can have prescribed proportions. The disc shape has length and width in a ratio of about 2:1, while the strip shape has an overall length about 4 times that of the disc, but a similar width to the disc for both the main body of the strip and the extension, and a similar length to the disc for the extension. The mould shapes are all of similar overall thickness of lesser order than the smallest of the above dimensions, with the thickness being in a ratio of about 1:7, say, with the disc width. For use in relation to a leg of an adult the disc mould shape has overall dimensions of about 40x20x3 mm and a range of reduced dimensions can be produced for use in relation to children.

It is to be noted that the strip shape is asymmetric and as illustrated is intended for use in relation to a right leg. Application to a left leg will involve a mirror image shaping.

Also. it will be noted that the mould shapes and their proportions are well suited to disposition in a compacted array with the discs effectively nested within the 'angle' of the strip, as shown, and this is advantageous in terms of economy in production of the tray.

As a last comment on the assembly _perse_, it is preferably covered, after the cast material is cured, with a protective plastics film or other suitable material which is readily peeled off or removed immediately prior to use of the moulds.

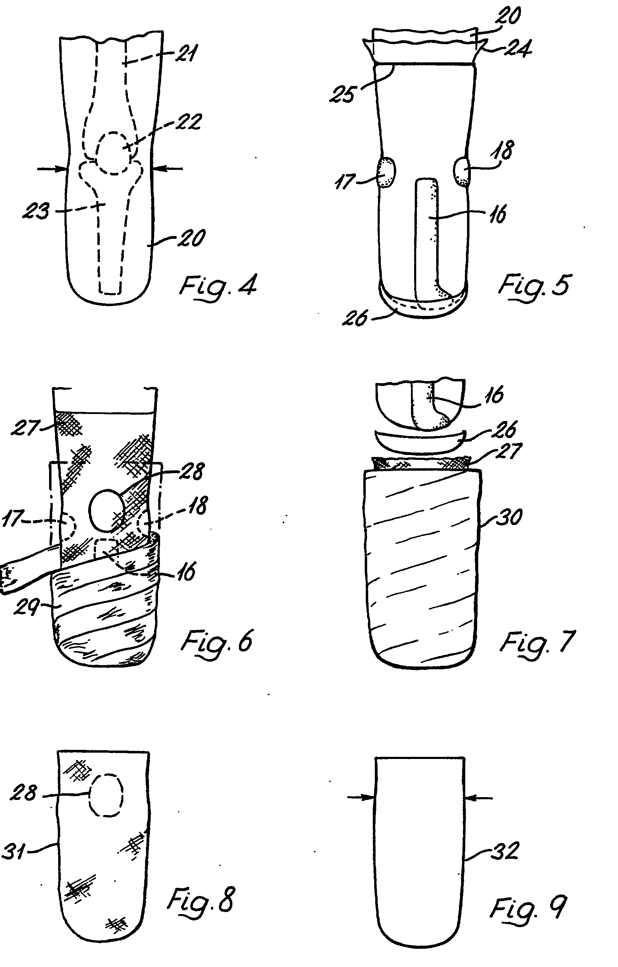

Turning to use of the moulds in the fitting procedure for the production of a prosthesis. Figure 4 shows a subject amputated right leg stump 20 and indicates in broken outline the lower end of the femur 21, the patella 22, and the shortened tibia 23. As a first step in the fitting procedure the width of the stump is measured with a suitable instrument at the level of the tibial condyles, the relevant width being indicated by the opposed horizontal arrows in Figure 4.

After this measurement, the stump is closely covered with a thin layer 24 of an appropriate plastics material such as self-adhesive polyester film to extend to above the knee where it is held by an elastic band 25, as shown in Figure 5. The mouldings 16-18 are then located in position, again as shown in Figure 5, on the covered stump where they are retained by natural adhesion. The discs are respectively located over the sides of the tibial condyles, with the disc lengths extending fore-and-aft. The strip moulding is located along the front of the stump over the tibia with the extended strip end lowermost and the extension directed inwardly of the stump. The upper end of the strip is to terminate just below the patella but this may require the strip to be cut to length at such end because the strip moulding length suitably allows for the maximum likely stump length following surgery.

After application of the mouldings a preformed cushioning cap 26 is placed over the lower end of the covered stump as shown in Figure 5, a wet thin sock 27 of knitted or similar form is located over the covered stump, mouldings and cap, and the sock is marked with an indelible pencil to indicate the position and extent of the patella such as by a circle 28, the sock and its marking being indicated in Figure 6.

Following this preparation of the stump, which includes rectification by way of the mouldings, the prepared stump is wrap cast with a plaster bandage 29 as indicated in Figure 6. The stump is held slightly bent at the knee during this operation and the prosthetist manipulates the tissue as during the conventional wrap casting technique.

When this casting and its curing is completed, the wrapped cast 30 is removed by withdrawal from the stump with the knee further bent as indicated by Figure 7, and the cap 26 removed. This cast is then itself used as a mould into which fresh plaster is poured. After curing of the second cast, the first cast is separated by cutting into the same to the depth of the sock where the casts are readily separated. The second cast 31 is exposed with a coarse surface finish and carries with it an adequate amount of the sock marking 28 to indicate the relative position of the patella as shown in Figure 8. This second cast is finished by removing, as appropriate, plaster to enhance the depressed shaping immediately below the patella and also behind the knee where the calf flares inwardly; the surface is smoothed overall; and it is checked in respect of its condylar width as shown by arrows again. The resultant replica 32 of the rectified stump is thereafter used in a conventional manner in the production of the cup in an artificial limb for the stump.

While the invention has been described with more specific reference to the drawings, this is by way of example and variation of the invention is possible within the scope of the appended claims. For example, application of the invention in relation to other forms of prosthesis than the patella tendon borne form, and also to limbs other than the leg, is possible as noted earlier. In addition to a set of mouldings of effectively standard shaping which will inevitably be applied in relation to a given prosthesis, one or more mouldings, suitably of relatively small disc and/or other shape, can be included in the assembly for location over sensitive areas of the stump detected during palpation by the prosthetist and individual to a particular patient. Also as noted earlier various materials are suitable for the precast moldings. The stump covering can be of various materials compatible with the skin and mouldings and it can be presented in the form of a bag or a protective film sheet such as of the widely available pvc self-adhesive type. The sock facilitates demarcation and separation of the two casts and is suitably of knitted synthetic fibre material. The cap is a conventional item of commercially available form, typically of coarse skeleton polyester foam material. Lastly, plaster is clearly an appropriate material for the casts for reason of its well-established suitability for such purposes, but alternatives can be used if desired.

## Claims

1. A method of making a limb prosthesis for an amputee patient which comprises rectifying the relevant amputated limb stump by the application thereto of at least one moulding of viscoelastic material precast to a predetermined shape, taking an impression of the rectified stump, and using the impression to determine the shape of a stump-receiving cup in said prosthesis.

2. A method according to Claim 1 wherein, before rectification, said stump is closely covered with a thin layer of protective material readily removable therefrom, and each said moulding is adhered to said layer.

3. A method according to Claim 1 or 2 wherein said impression is taken by wrapping casting material in bandage form around said rectified stump and thereafter removing the resultant wrapped cast when set.

4. A method according to Claim 3 wherein said socket shape is determined from said impression by pouring further casting material into said wrapped cast to form a replica of said rectified stump, removing said cast to expose said replica when set, and forming said socket on said replica.

5. A method according to Claim 4 wherein said bandage form and further casting materials each comprise plaster, and said rectified stump is covered with a sock of material which becomes bonded and removable with said wrapped cast subsequently to demarcate the same from said replica.

6. A method according to Claim 5 wherein a positional reference marking for said stump is applied to said sock which marking is incorporated into said wrapped cast and thereafter conveyed, at least in part, to said replica.

7. A method according to any preceding claim for making a leg prosthesis of patella borne form for a below-the-knee amputee patient, wherein said rectifying comprises applying one said moulding of disc shape over one side of the condylar protuberances, applying a similar moulding over the other side of said protuberances, and applying a further said moulding of strip shape longitudinally over the front of the tibia.

8. A method according to Claim 7 wherein, at the same time as said rectifying and before application of said sock, a cap of cushioning material is located over the free end of said stump.

9. For use in accordance with the method according to any preceding claim, an assembly of separate mouldings of viscoelastic material individually precast to a respective predetermined shape.

10. An assembly according to Claim 9 wherein said mouldings are releasably formed in respective dished zones of a common support.

11. An assembly according to Claim 10 wherein said support is stackable with other assemblies of like form.

12. An assembly according to Claim 9,10 or 11 for use in a method according to Claim 7 or 8, comprising three said mouldings including two of like oval disc shape in plan, and another of strip shape in plan having one end portion transversely extended along one side, each such shape being of decreasingly tapered flared form towards its periphery in cross-section.

13. An assembly according to Claim 12 wherein said transverse extension is of generally progressively increasing width towards the free end of said one end portion.

14. An assembly according to Claim 12 or 13 wherein said disc shape has its overall length and width in a ratio of about 2:1; said strip shape has an overall length about 4 times that of said disc. but an overall width for each of the main body of said strip and said extension similar to that for said disc, and a similar overall length for said extension to said disc; and each said shape has an overall thickness in a ratio of about 1:7 with said disc width.

15. An assembly according to Claim 14 wherein said disc shape has overall dimensions of about 40*20*3 mm.

16. An assembly according to any one of Claims 12-15 wherein said three mouldings are mutually disposed in a nested array with said disc shapes located alongside successive portions of the main body of said strip shape in the angle formed between such body and the associated extension.

## Patentansprüche

1. Verfahren zum Herstellen einer Gliedprothese für einen amputierten Patienten, umfassend das Ausrichten des relevanten amputierten Gliedstumpfes durch Anbringung mindestens eines Formteils aus viscoelastischem Material daran, das in einer vorbestimmten Form vorgefertigt ist, Abnehmen eines Abdruckes des ausgerichteten Stumpfes und Verwenden des Abdruckes zum Festlegen der Form einer den Stumpf aufnehmenden Pfanne bzw. eines Lagers für den Stumpf in der Prothese.

2. Verfahren nach Anspruch 1, bei dem vor der Rektifikation der Stumpf dicht mit einer dünnen Schicht eines schützenden Materials abgedeckt wird, das davon leicht entfernbar ist, und wobei das bzw. die Formteile an dieser Schicht angebracht werden.

3. Verfahren nach Anspruch 1 oder 2, bei dem der Abdruck dadurch abgenommen wird, daß man das Formmaterial in Form einer Bandage um den ausgerichteten Stumpf herumwickelt und danach den entstandenen umwickelten Abguß entfernt, wenn er formfest geworden ist.

4. Verfahren nach Anspruch 3, bei dem die Sockelform vom Abdruck dadurch abgenommen wird, daß man zusätzliches Form- bzw. Gußmaterial in den umwickelten Abguß hineinfüllt, um eine Replik des ausgerichteten Stumpfes zu bilden, und wobei man den Abguß entfernt, um die Replik freizulegen, wenn sie formfest geworden ist, und daß man den Sockel an der Replik ausbildet bzw. befestigt.

5. Verfahren nach Anspruch 4, bei dem die Bandagenform und die weiteren Form- bzw. Abgußmaterialien jeweils Gips umfassen, und daß der ausgerichtete Stumpf mit einem Materialstrumpf überzogen wird, der sich mit dem umwickelten Abguß verbindet und damit entfernbar ist, um diesen anschließend von der Replik abzugrenzen.

6. Verfahren nach Anspruch 5, bei dem eine stellungsmäßige Bezugsmarkierung für den Strumpf am Stumpf angebracht wird, und wobei diese Markierung im umwickelten Abguß angeordnet und anschließend, zumindest teilweise, auf die Replik übertragen wird.

7. Verfahren nach irgendeinem der vorstehenden Ansprüche zum Herstellen einer Beinprothese patellaartiger Form für einen unter dem Knie amputierten Patienten, bei dem die Ausrichtung umfaßt, daß man ein Formteil in Scheibenform über eine Seite der condylären Vorsprünge anbringt, und wobei man ein gleiches Formteil über die andere Seite der Vorsprünge anbringt, und wobei man ein weiteres Formteil in Streifenform längs über die Vorderseite der Tibia anbringt.

8. Verfahren nach Anspruch 7, bei dem zur gleichen Zeit des Ausrichtens und vor der Anbringung des Sockens bzw. Strumpfes, eine Kappe aus Dämpfungsmaterial über das freie Ende des Stumpfes angeordnet wird.

9. Eine Anordnung getrennter Formteile aus viscoelastischem Material, die individuell in einer entsprechenden vorbestimmten Form vorgefertigt sind, zur Verwendung in Übereinstimmung mit dem Verfahren gemäß irgendeinem vorstehenden Anspruch.

10. Anordnung nach Anspruch 9, bei der die Formteile wieder lösbar in entsprechend konkav gewölbten Bereichen eines gemeinsamen Lagers ausgebildet sind.

11. Anordnung nach Anspruch 10, bei der das Lager mit anderen Anordnungen gleicher Form stapelbar ist.

12. Anordnung nach Anspruch 9, 10 oder 11 zur Verwendung bei einem Verfahren nach Anspruch 7 oder 8, umfassend drei Formteile, einschließlich zwei gleicher, ovaler Scheibenform in der Aufsicht, und eines weiteren in Streifenform in der Draufsicht, das einen Endteil besitzt, der sich entlang einer Seite quer dazu erstreckt, wobei jede dieser Formen im Querschnitt in Richtung auf ihren Außenumfang eine abnehmend zugespitzte, sich konisch erweiternde Form aufweist.

13. Anordnung nach Anspruch 12, bei der die querverlaufende Erstreckung eine generell allmählich zunehmende Breite in Richtung zum freien Ende des einen Endteils aufweist.

14. Anordnung nach Anspruch 12 oder 13, bei der die Scheibenform hinsichtlich ihrer Gesamtlänge und Breite ein Verhältnis von etwa 2:1 aufweist, und wobei die Streifenform eine Gesamtlänge aufweist, die etwa das Vierfache der Scheibe beträgt, jedoch eine Gesamtbreite für jeden der Hauptkörper des Streifens und der Erstreckung, die der der Scheibe entsprechen, sowie eine gleiche Gesamtlänge für die Erstreckung zur Scheibe hin, und wobei jede dieser Formen eine Gesamtdicke im Verhältnis von etwa 1:7 zur Scheibenbreite besitzt.

15. Anordnung nach Anspruch 14, bei der die Scheibenform Abmessungen von etwa 40*20*3 mm besitzt.

16. Anordnung nach irgendeinem der Ansprüche 12 bis 15, bei der drei Formteile gegenseitig zueinander in einer geschachtelten Anordnung angeordnet sind, wobei die Scheibenformen längsseits aufeinanderfolgender Teile des Hauptkörpers der Streifenform in einem Winkel angeordnet sind, der zwischen diesem Körper und der zugehörigen Erstreckung ausgebildet ist.

## Revendications

1. Procédé pour réaliser une prothèse de membre pour un patient amputé, ce procédé comprenant la rectification du moignon de membre amputé en cause par application sur ce moignon d'au moins un moulage en une matière viscoélastique prémoulée à une forme déterminée, la prise d'une empreinte du moignon rectifié, et l'utilisation de l'empreinte pour déterminer la forme d'une cuvette destinée à loger ou recevoir le moignon dans ladite prothèse.

2. Procédé selon la revendication 1 dans lequel, avant la rectification, ledit moignon est étroitement recouvert d'une mince couche d'une matière protectrice pouvant en être facilement enlevée, et l'on fait adhérer chacun desdits moulages à ladite couche.

3. Procédé selon la revendication 1 ou 2, dans lequel ladite empreinte est obtenue par enveloppement de la matière, destinée à former le moulage, sous forme de bandage autour dudit moignon rectifié, puis enlèvement, après sa prise ou durcissement, du moulage résultant de l'enveloppement.

4. Procédé selon la revendication 3, dans lequel ladite forme de cuvette de base est déterminée, à partir de ladite empreinte, par versement d'une matière supplémentaire à mouler dans ledit moule obtenu par enveloppement, pour former une réplique dudit moignon rectifié, par enlèvement dudit moule pour exposer ou mettre à nu ladite réplique une fois durcie, et par formation de ladite cuvette de base sur ladite réplique.

5. Procédé selon la revendication 4, dans lequel ledit produit en forme de bandage et les autres matières pour moulage comprennent chacune du plâtre, et ledit moignon rectifié est recouvert d'une matière en forme de chaussette qui se fixe sur ledit moulage obtenu par enveloppement et peut en être ensuite enlevée pour en assurer la séparation de démarquage d'avec ladite réplique.

6. Procédé selon la revendication 5, dans lequel une marque de référence de position dudit moignon est appliquée sur la chaussette, cette marque étant incorporée au moulage obtenu par enveloppement et étant ensuite transférée, au moins en partie, sur ladite réplique.

7. Procédé selon l'une quelconque des revendications précédentes pour réaliser une prothèse de jambe de forme portée par la rotule pour un patient amputé au-dessous du genou, procédé dans lequel la rectification comprend l'application d'une pièce moulée en forme de disque sur un côté des protubérances correspondant aux condyles, l'application d'un moulage semblable sur l'autre côté desdites protubérances, et l'application d'un moulage supplémentaire en forme de bande longitudinalement sur l'avant du tibia.

8. Procédé selon la revendication 7 dans lequel, en même temps que la rectification et avant l'application de la chaussette, on place un capuchon de matière de rembourrage sur l'extrémité libre dudit moignon.

9. Pour servir selon le procédé d'après l'une quelconque des revendications précédentes, un ensemble de moulages séparés en une matière viscoélastique, chacun prémoulé individuellement à une forme respective prédéterminée.

10. Ensemble selon la revendication 9, dans lequel lesdits moulages sont formés, de manière amovible, dans des zones respectives en creux d'un support commun.

11. Ensemble selon la revendication 10, dans lequel ledit support est empilable avec d'autres ensembles de forme analogue.

12. Ensemble selon la revendication 9, 10 ou 11, destiné à servir dans un procédé selon la revendication 7 ou 8, comprenant trois desdits moulages comprenant deux moulages en forme de disques ovales semblables en plan et un autre moulage en forme de bande en plan ayant une partie terminale qui s'étend transversalement le long d'un côté, chacune de ces formes ayant une forme s'évasant par effilement qui diminue progressivement vers sa périphérie, en coupe.

13. Ensemble selon la revendication 12, dans lequel ledit prolongement transversal a une largeur qui augmente de façon généralement progressive vers l'extrémité libre de ladite partie terminale.

14. Ensemble selon la revendication 12 ou 13, dans lequel ladite forme de disque présente entre sa longueur et sa largeur totales, un rapport d'environ 2:1; ladite forme en bande a une longueur totale représentant environ quatre fois celle du disque, mais une largeur globale pour le corps ou partie principale de ladite bande et pour ledit prolongement, semblable à la largeur dudit disque, et une longueur globale dudit prolongement semblable à celle dudit disque; et chacune desdites formes a une épaisseur globale présentant un rapport d'environ 1:7 avec ladite largeur de disque.

15. Ensemble selon la revendication 14, dans lequel ladite forme de disque a des dimensions globales d'environ 40*20*3 mm.

16. Ensemble selon l'une quelconque des revendications 12 à 15, dans lequel lesdits trois moulages sont mutuellement disposés en un réseau imbriqué, lesdites formes de disques étant situées le long de parties successives du corps principal de ladite forme de bande, dans l'angle formé entre le corps de la bande et le prolongement qui lui est associé.

EP 0 261 884 B1

Fig.1

Fig.2

Fig.3

EP 0 261 884 B1

Fig. 4

Fig. 5

Fig. 6

Fig. 7

Fig. 8

Fig. 9